# EUROPÄISCHE PATENTSCHRIFT

(11) **EP 1 402 818 B1**
(45) Veröffentlichungstag und Bekanntmachung des Hinweises auf die Patenterteilung: **31.08.2005**
(21) Anmeldenummer: 03405661.4
(22) Anmeldetag: 10.09.2003
(51) Int. Cl.: A61B 6/14, G03B 42/04

(54) **Halter für digitale Sensoren für die Zahnmedizin**
Holder for digital sensors in dentistry
Support de détecteur digitale médico-dentaire

(30) Priorität: 30.09.2002 CH 16322002
(43) Veröffentlichungstag der Anmeldung: 31.03.2004
(60) Teilanmeldung: 05013875.9
(73) Patentinhaber: Kerrhawe SA, 6934 Bioggio (CH)
(72) Erfinder: Kilcher, Beat, 6935 Bosco-Luganese (CH); Da Rold, Marco, 6951 Odogno (CH)
(74) Vertreter: AMMANN PATENTANWÄLTE AG BERN

(56) Entgegenhaltungen:
- CA-A- 2 324 871
- FR-A- 1 088 070
- US-A- 1 571 145
- US-A- 1 576 477
- US-A- 2 688 096
- US-A- 4 057 732
- US-A- 4 965 885
- US-A- 5 799 058
- US-B1- 6 203 195
- US-B1- 6 343 875

## Beschreibung

Die vorliegende Erfindung bezieht sich auf einen Halter-Satz für digitale Sensoren für die Zahnmedizin.

Bis vor kurzem wurden die Röntgenaufnahmen auf Röntgenfilmen fixiert, die belichtet und später entwickelt wurden. Solche Röntgenfilme haben bestimmte, genormte Formate, so dass die Halter, bzw. deren Klemmteil zur Aufnahme der Röntgenfilme, normierte Grössen aufweisen. Probleme bei solchen Röntgenfilmhalterungen ergaben und ergeben sich in erster Linie daraus, die Aufnahme von verschiedenen Zahnteilen, d.h. die Aufnahme von Bissflügel sowie anderen Teilen der Zähne mit möglichst wenig verschiedenen Haltern durchführen zu können, wobei die Filmformate meist entweder quer oder längs eingeklemmt werden.

In neuerer Zeit wurde eine neue Abbildungstechnik entwickelt, indem digitale Bildsensoren über Kabel direkt an einen Computer angeschlossen werden, um die Röntgenaufnahmen direkt darauf zu visualisieren oder zu speichern.

Im Gegensatz zu den Röntgenfilmen besitzen die verschiedenen Produkte von digitalen Sensoren verschiedene Dimensionen, sowohl in der Länge und Breite als auch in der Dicke. Hinzu kommt, dass solche Sensoren ein Kabel aufweisen, wodurch die lagerichtige Halterung solcher Sensoren problematisch ist.

Es sind bereits eine Anzahl von Sensorhaltern bekannt, so gemäss US-A-6 203 195, die einen Sensorhalter offenbart, bei dem ein Halterarm ausziehbar ist, um verschiedene Formate halten zu können. Obwohl hier bereits ein Ansatz vorliegt, verschiedene Formate zu verwenden, ist die universale Anwendung eines solchen Halters durch dessen Konstruktion eingeschränkt.

Aus der WO/49 945 ist ein weiterer Sensorhalter bekannt, bei dem ein Halterarm ausziehbar ist, um Sensoren mit verschiedenen Dimensionen zu befestigen. Auch hier gestattet die Konstruktion nicht die universale Anwendung von verschiedensten Sensoren.

CA-A1-2 324 871 offenbart einen Aufbissblock für Sensoren, mit welchem Sensoren mit unterschiedlichen Abmessungen zwischen zwei Klemmplatten eingespannt werden können.

US-B1-6 203 195 offenbart einen Halter für Sensoren bei dem die Sensorhalterung direkt am Aufbiss angeordnet ist und jeweils ein beweglicher Arm gegen einen am Aufbiss angeordneten Anschlag verschiebbar ist.

US-B1-6 343 875 offenbart einen Halter für digitale Sensoren, bei welchem der Sensorhalter starre Klemmarme aufweist und daher für jedes unterschiedliche Format ein anderer Sensorhalter aufgesetzt werden muss.

Es ist von diesem Stand der Technik ausgehend Aufgabe der vorliegenden Erfindung, einen Satz mit einer Mindestanzahl von Sensorenhaltern zu schaffen, die Röntgenaufnahmen mit sämtlichen sich auf dem Markt befindlichen digitalen Sensoren im Frontzähne- und Seitenbereich ermöglichen, d.h. im periapikalen Gewebe sowie horizontale und vertikale Bissflügelaufnahmen mit einerseits guter Ausrichtmöglichkeit der Röntgenröhre auf die Sensoren und andererseits maximalem Komfort für den Patienten. Diese Aufgabe wird mit dem Haltersatz gemäss Patentanspruch 1 gelöst. Die weiteren Ansprüche geben jeweils bevorzugte Ausführungsformen an.

Die Erfindung wird im Folgenden anhand von Zeichnungen von Ausführungsbeispielen näher erläutert.
- Fig. 1: zeigt ein erstes Ausführungsbeispiel eines Klemmteils eines Halters für periapikale Aufnahmen,
- Fig. 2: zeigt einen Ausschnitt aus dem Klemmteil von Fig. 1,
- Fig. 3: zeigt das Klemmteil von Fig. 1 mit einem vertikal angeordneten Sensor,
- Fig. 4: zeigt ein zweites Ausführungsbeispiel eines Klemmteils für einen Halter für Bissflügelaufnahmen, ohne Abdeckung,
- Fig. 5: zeigt das Klemmteils von Fig. 4 aus der entgegengesetzten Sicht,

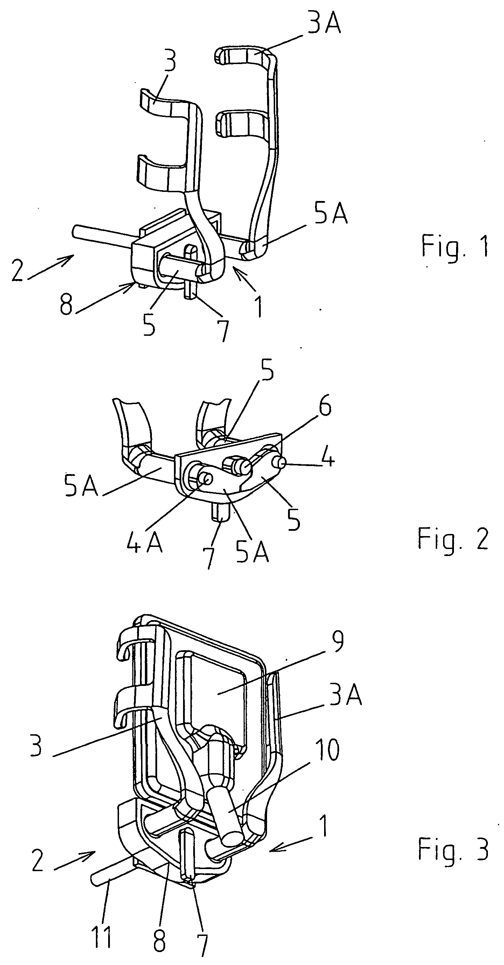

In den Figuren 1 bis 3 ist ein erstes Ausführungsbeispiel einer Sensorklemme an einem Halter dargestellt, bei welchem die beiden Arme mit den Halteleisten selbstzentriert bewegt werden. Die Sensorklemme 1 von Halter 2 weist zwei Arme 3 und 3A auf, die je um eine Achse 4 und 4A schwenkbar sind und je einen abgewinkelten Hebel 5, 5 A aufweisen, auf die eine Nockenwelle 6 wirkt derart, dass die Hebel in Fig. 2 nach unten und damit die Arme nach einwärts geschwenkt werden. Auf die Nockenwelle wirkt ein Arretierhebel 7, um die Arme zu entriegeln, wodurch sie in die Offen-Stellung schwenken. Die Nockenwelle mit den Hebeln ist in einem Gehäuse 8 angeordnet.

Dieser Halter eignet sich besonders für periapikale Frontzahnaufnahmen, und man erkennt aus Fig. 3, dass der digitale Sensor 9 mit Kabel 10 so seitlich eingespannt und die Arme derart gestaltet sind, dass seitlich wenig Platz eingenommen wird. Ausserdem erkennt man aus Fig. 3, dass die Sensorklemme nicht über den Sensor 9 hinausragt.

Aus den Figuren 1 oder 3 ist ein Teil 11 des Halters 2 ersichtlich, der im Prinzip ähnlich demjenigen Halter gemäss EP-B-397 599 aufgebaut ist. Dabei sind sowohl der Aufbiss als auch der gekröpfte Indikatorstab dieselben oder ähnlich wie bei diesem europäischen Patent, während die federnde Röntgenfilm-Klemme durch das Klemmteil 1 von Figur 1 ersetzt ist.

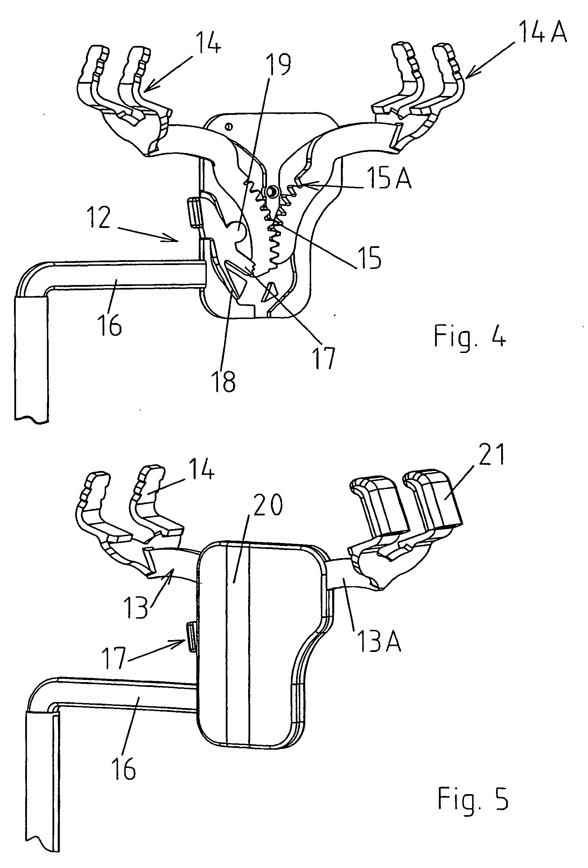

In den Fig. 4 und 5 ist ein Halter für die Bissflügelaufnahmen dargestellt, wobei die dargestellte Sensorklemme anstelle der Röntgenfilmklemme angeordnet ist. Röntgenfilmhalter für Bissflügelaufnahmen sind bestens bekannt und werden u.a. auch von der Anmelderin seit langem vertrieben. Daher sind die in den Fig. 4 und 5 nicht oder unvollständig dargestellten Teile, wie der abgekröpfte Indikatorstab, nicht näher beschrieben. Analog zur Sensorklemme gemäss den Fig. 1 - 3 bewegen sich dessen Arme ebenfalls symmetrisch, so dass der Sensor bezüglich des Indikatorstabes bzw. der Röntgenröhre stets gleich ausgerichtet ist, unabhängig von der Dimension des Sensors.

In Fig. 4 ist die Sensorklemme 12 ohne Gehäuse und schematisch dargestellt, und man erkennt die beiden Arme 13 und 13A mit den Greifern 14 und 14A. An den Armen schliesst sich ein gekrümmtes Teil mit Zahnstange 15 und 15A an, wobei beide Zahnstangen ineinander greifen. In Fig. 4 ist ferner ein Teil des Indikatorstabes 16 ersichtlich. Die Bewegung der Zahnstangen wird durch eine Sperre 17 beeinflusst, wobei diese Sperre selbsthemmend ist, so dass die Arme in jeder Stellung bleiben, in die sie gerückt wurden. Ausserdem besitzt die Sperre eine Rückfeder 18, so dass das eine Ende der Sperre stets auf eine gekrümmte Zahnstange wirkt, um die Selbsthemmung zu bewirken. Die Sperre dreht um Drehpunkt 19.

Die Lösung mit den gekrümmten Zahnstangen erlaubt es, den ganzen Spreiz- und Arretiermechanismus sehr flach zu gestalten, wodurch die Aufbissplatte 20, die durch das Gehäuse des Mechanismus geformt ist, sehr flach gehalten werden kann, siehe Fig. 5, wodurch der ganze Halter für den Patienten angenehmer ist als vorbekannte Halter. Die Arme, bzw. Klemmbacken mit den Greifern sind so gestaltet, dass sie jede Sensorgeometrie aufnehmen können, d.h. sie weisen eine prismatische Form auf. Dies geht gut aus Fig. 5 hervor.

In einer Ausführungsvariante sind auf den Greifern 13, 13A Gummipuffer 21 aufgesteckt. Dazu ist es zweckmässig, dass die Greifer eine Struktur haben, die solche Gummipuffer zurückhalten.

Allen Sensor-Klemmen gemäss der Erfindung ist gemeinsam, dass mit Ihnen die digitalen Sensoren ohne Probleme mit den hygienischen Beuteln oder sonstigen Schutzhüllen genau gehalten werden können und dass zwischen dem Sensor und der Röntgenröhre kein anderer Kunststoff vorhanden ist. Auch geht aus der Beschreibung klar hervor, dass Sensoren mit jeglichen Abmessungen gehalten werden können. Ferner ist aus der Beschreibung ersichtlich, dass die Sensoren immer gefasst werden und dass sie nicht in die Klemme hineingepresst werden, da die Arme immer geöffnet werden können, um den Sensor aufzunehmen, um ihn anschliessend zu fassen.

## Patentansprüche

1. Halter-Satz für digitale Sensoren für die Zahnmedizin, mit einem Halter für periapikale Aufnahmen von Seitenzähnen 5 und Frontzähnen und einem Halter für Bissflügelaufnahmen, mit je einem Aufbiss und einem gekröpften Indikatorstab, wobei jeder Halter (2, 12) zwei symmetrisch bewegliche Arme (3, 3A; 13, 13A) aufweist, die derart angeordnet und miteinander verbunden sind, dass für jede Aufnahmeart ein Sensor (9) unabhängig von seinen Abmessungen jeweils selbstzentriert von den zwei Armen gefasst wird und bezüglich des Indikatorstabes stets gleich ausgerichtet ist.

2. Halter-Satz nach Anspruch 1, **dadurch gekennzeichnet, dass** die selbstzentrierende Bewegung 15 der Arme (3, 3A) des Halters für periapikale Aufnahmen (2) durch daran angeordnete, abgewinkelte Hebel (5, 5A) ermöglicht wird, auf die eine schwenkbare Nockenwelle (6) wirkt.

3. Halter-Satz nach Anspruch 2, **dadurch gekennzeichnet, dass** 20 die Nockenwelle (6) mit einem Arretierhebel (7) in Wirkverbindung steht.

4. Halter-Satz nach Anspruch 1, **dadurch gekennzeichnet, dass** die selbstzentrierende Bewegung der Arme (13, 13A) des Halters für Bissflügelaufnahmen (12) durch ein an jedem Arm angeordnetes, gekrümmtes Teil mit Zahnstange (15, 15A) ermöglicht wird, wobei die Zahnstangen miteinander im Eingriff stehen.

5. Halter-Satz nach Anspruch 4, **dadurch gekennzeichnet, dass** der Halter für Bissflügelaufnahmen (12) eine unter Federwirkung (18) stehende, auf mindestens eine Zahnstange wirkende Sperre (17) aufweist.

## Claims

1. Holder set for digital sensors for dentistry, including a holder for periapical radiographs of lateral and anterior teeth and a holder for bitewing radiographs, each comprising a bite piece and a cranked indicator rod, each holder (2, 12) comprising two symmetrically displaceable arms (3, 3A; 13, 13A) that are arranged and connected to each other such that for every type of radiograph, a sensor (9) is seized independently of its format in a self-centred manner by the two arms and always aligned with respect to the indicator rod in the same way.

2. Holder set according to claim 1, **characterized in that** the self-centring movement of the arms (3, 3A) of the holder for periapical radiographs (2) is provided by angled levers (5, 5A) arranged thereon, the levers being actuated by a pivotable camshaft (6).

3. Holder set according to claim 2, **characterized in that** the camshaft (6) is operatively connected to a locking lever (7).

4. Holder set according to claim 1, **characterized in that** the self-centring movement of the arms (13, 13A) of the holder for bitewing radiographs (12) is provided by a respective curved portion arranged on each arm and including a toothed rack (15, 15A), the toothed racks meshing with each other.

5. Holder set according to claim 4, **characterized in that** the holder for bitewing radiographs (12) comprises a catch (17) actuated by a spring (18) and acting upon one of the toothed racks at least.

## Revendications

1. Jeu de supports pour capteurs numériques pour la dentisterie, composé d'un support pour radiographies péri-apicales de dents latérales et frontales et d'un support pour radiographies interproximales, comprenant chaque fois une pièce à mordre et une tige indicatrice coudée, chacun des supports (2, 12) présentant deux bras (3, 3A; 13, 13A) déplaçables en symétrie qui sont agencés et reliés entre eux de telle manière que pour chaque type de radiographie, un capteur (9) est chaque fois saisi de manière autocentrante indépendamment de son format par les deux bras et toujours orienté par rapport à la tige indicatrice de la même manière.

2. Jeu de supports selon la revendication 1, **caractérisé en ce que** le mouvement autocentrant des bras (3, 3A) du support pour radiographies péri-apicales (2) est produit par des leviers coudés (5, 5A) agencés sur le support et actionnés par un arbre à came (6) pivotant.

3. Jeu de supports selon la revendication 2, **caractérisé en ce que** l'arbre à came (6) est fonctionnellement relié à un levier de blocage (7).

4. Jeu de supports selon la revendication 1, **caractérisé en ce que** le mouvement autocentrant des bras (13, 13A) du support pour radiographies interproximales (12) est produit par des parties courbées de chaque bras qui sont munies de crémaillères (15, 15A), les crémaillères s'engrenant.

5. Jeu de supports selon la revendication 4, **caractérisé en ce que** le support pour radiographies interproximales (12) comporte un cliquet d'arrêt (17) soumis à l'action d'un ressort (18) et agissant sur l'une au moins des crémaillères.
